# FASCICULE DE BREVET EUROPEEN

(11) **EP 3 433 323 B1**
(45) Date de publication et mention de la délivrance du brevet: **15.07.2020**
(21) Numéro de dépôt: 17715239.4
(22) Date de dépôt: 17.03.2017
(51) Int. Cl.: C09D 4/00, B65D 90/04, C07C 21/18, C09D 171/00, C08G 18/48, F17C 1/00

(54) **RECIPIENT POUR LE STOCKAGE D'UNE COMPOSITION COMPRENANT DU TETRAFLUOROPROPENE ET MÉTHODE DE STOCKAGE DE CELLE-CI**
BEHÄLTER ZUR LAGERUNG EINER ZUSAMMENSETZUNG MIT TETRAFLUORPROPEN UND LAGERUNGSVERFAHREN DAFÜR
CONTAINER FOR STORING A COMPOSITION COMPRISING TETRAFLUOROPROPENE AND METHOD FOR STORING SAME

(30) Priorité: 22.03.2016 FR 1652437
(43) Date de publication de la demande: 30.01.2019
(73) Titulaire: Arkema France, 92700 Colombes (FR)
(72) Inventeur: BOUSSAND, Béatrice, 69491 Pierre-Benite Cedex (FR); RACHED, Wissam, 69630 Chaponost (FR); WENDLINGER,Laurent, 69510 Soucieu En Jarrest (FR)
(74) Mandataire: Leca, François Michel
(86) Numéro de dépôt international: PCT/FR2017/050618
(87) Numéro de publication internationale: WO 2017/162962

(56) Documents cités:
- EP-A1- 0 006 334
- WO-A1-2014/197215
- US-A- 4 442 246
- US-A1- 2015 051 426

## Description

### Domaine technique

La présente invention concerne une méthode de stockage d'une composition comprenant du tetrafluoropropene. La présente invention concerne aussi un récipient pour stocker une composition comprenant du tetrafluoropropene. En particulier, la présente invention concerne un récipient et une méthode pour stocker de manière stable une composition comprenant du tetrafluoropropene.

### Arrière-plan technologique de l'invention

Au cours des dernières années, le 2,3,3,3-tetrafluoropropene (CF₃CF=CH₂, ci-après également HFO-1234yf) a attiré l'attention en tant que nouveau réfrigérant pour remplacer des réfrigérants tels que par exemple les chlorofluorocarbures (CFC), les hydrochlorofluorocarbures (HCFC) ou les hydrofluorocarbures (HFC), qui sont des gaz à effet de serre. De manière générale, les composés du type hydrochloropropene, hydrofluoropropene peuvent être instables et générer la formation de coproduits en présence d'eau, d'acide ou d'oxygène. On connaît notamment par US 9,228,128 une méthode de stabilisation du 1,1,2,3-tetrachloropropene en présence de morpholine ou d'un dérivé de celle-ci ou de phosphate de trialkyle.

Le HFO-1234yf est quant à lui généralement stocké à l'état gazeux et/ou à l'état liquide et transporté dans un récipient fermé sous pression. Dans certaines conditions, le HFO-1234yf peut se dégrader et/ou polymériser en présence d'oxygène. US2015/0051426 décrit notamment le stockage du HFO-1234yf dans un conteneur dans lequel la concentration en oxygène est maintenue en dessous de 1000 ppm en volume pour empêcher sa dégradation et sa polymérisation.

La présence de dépôts solides liés à la dégradation du HFO-1234yf lors de son stockage ou de son transport peut empêcher son utilisation ultérieure en tant que réfrigérant. En outre, en cas de présence d'un stabilisateur dans la composition, il est nécessaire d'enlever le stabilisateur avant l'utilisation du HFO-1234yf ce qui complexifie sa mise en œuvre et augmente les risques liés à la présence résiduelle du stabilisant.

US 4 442 246, EP 0 006 334 et WO 2014/197215 décrivent des récipients en métal et comprenant une surface intérieure recouverte par une résine à base de polyol or polyéther. Toutefois ces documents ne décrivent pas de compositions comprenant du tétrafluoropropene.

Il y a donc un besoin pour la mise en œuvre d'une méthode de stockage permettant d'éviter les inconvénients des méthodes connues.

### Résumé de l'invention

Selon un premier aspect, l'invention fournit un récipient contenant une composition comprenant du tetrafluoropropene, ledit récipient étant en métal et comprenant une surface intérieure, ladite surface intérieure en contact avec ladite composition étant au moins partiellement recouverte par une résine de type polyéther ou polyol, avantageusement ladite composition comprend au moins 15% en poids de tetrafluoropropene sur base du poids total de la composition.

Selon un mode de réalisation préféré, la résine de type polyéther ou polyol est issue de monomères comprenant un groupement fonctionnel oxirane ou phénol.

De préférence, la résine de type polyéther ou polyol est issue de monomères comprenant un motif siloxirane.

En particulier, la résine de type polyéther ou polyol est issue de monomères comprenant un motif siloxirane de formule (I) 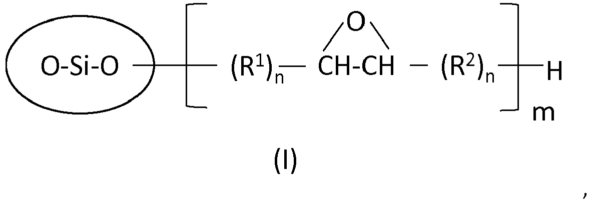 dans laquelle R¹ et R² sont indépendamment l'un de l'autre, et indépendamment pour chaque unité n et m, un groupement C₆-C₁₈ aryle, C₁-C₂₀ alkyle, C₃-C₂₀ cycloalkyle, C₃-C₂₀ cycloalkényle, C₂-C₂₀ alkényle, carbonyle de formule R³-C(O)-R⁴ , ester de formule R³-C(O)-O-R⁴, éther de formule R³-O-R⁴; une amine de formule R³-N-R⁴, R² pouvant également être un groupement aldéhyde de formule R³-C(O)-H ; R³ et R⁴ étant choisis indépendamment l'un de l'autre parmi un groupement C₆-C₁₈ aryle, C₁-C₂₀ alkyle, C₃-C₂₀ cycloalkyle, C₃-C₂₀ cycloalkényle ou C₂-C₂₀ alkényle ; m est un nombre entier de 1 à 30, avantageusement de 1 à 20, de préférence de 5 à 10 ; et
n est indépendamment pour R¹ et R² un nombre entier de 1 à 30, avantageusement de 1 à 20, de préférence de 5 à 10.

Selon un autre mode de réalisation préféré, la résine de type polyéther ou polyol est issue de condensats d'un composé **A** avec un composé **B**, le composé **A** étant un composé phénol substitué ou non et le composé **B** étant un composé de formule R¹C(O)R² dans laquelle R¹ et R² sont indépendamment hydrogène, C₁-C₂₀ alkyle, C₆-C₂₀ aryle, C₃-C₂₀ cycloalkyle, C₂-C₂₀ alkényle.

De préférence, le composé **A** est le phénol C₆H₅OH et le composé **B** est le formaldéhyde.

Selon un mode de réalisation préféré, au moins 90% de ladite surface intérieure en contact avec ladite composition est recouverte par ladite résine de type polyéther ou polyol, avantageusement au moins 95% de ladite surface intérieure en contact avec ladite composition est recouverte par ladite résine de type polyéther ou polyol, de préférence au moins 98% de ladite surface intérieure en contact avec ladite composition est recouverte par ladite résine de type polyéther ou polyol, en particulier au moins 99% de ladite surface intérieure en contact avec ladite composition est recouverte par ladite résine de type polyéther ou polyol, plus particulièrement toute la surface intérieure du récipient en contact avec ladite composition est recouverte par ladite résine de type polyéther ou polyol.

Selon un mode de réalisation préféré, le récipient est en acier.

Selon un mode de réalisation préféré, le récipient est en acier au carbone.

Selon un mode de réalisation préféré, la composition comprend moins de 10000 ppm en poids d'eau sur base du poids total de la composition, avantageusement moins de 5000 ppm en poids d'eau sur base du poids total de la composition, de préférence moins de 1000 ppm en poids d'eau sur base du poids total de la composition, en particulier de 0,1 ppm à 100 ppm en poids d'eau sur base du poids total de la composition, plus particulièrement de 1 à 20 ppm en poids d'eau sur base du poids total de la composition.

Selon un mode de réalisation préféré, la composition a une teneur en acide, calculée en équivalent d'acide chlorhydrique, inférieure à 100 ppm en poids sur base du poids total de la composition, avantageusement inférieure à 50 ppm en poids sur base du poids total de la composition, de préférence inférieure à 10 ppm en poids sur base du poids total de la composition, en particulier de 0,01 ppm à 2 ppm en poids sur base du poids total de la composition.

Selon un mode de réalisation préféré, la composition est composée d'une phase gazeuse et d'une phase liquide.

Selon un mode de réalisation préféré, la composition comprend une teneur en air gazeux de 0,1% à 5% en volume sur base du volume total de la phase gazeuse, avantageusement de 0,5% à 3% en volume sur base du volume total de la phase gazeuse, de préférence de 0,01% à 2% en volume sur base du volume total de la phase gazeuse.

Selon un mode de réalisation préféré, la composition comprend au moins 90% en poids de tetrafluoropropene sur base du poids total de la composition, avantageusement au moins 95% en poids sur base du poids total de la composition, de préférence au moins 98% en poids sur base du poids total de la composition, en particulier au moins 99,5% en poids sur base du poids total de la composition.

Selon un mode de réalisation préféré, le tetrafluoropropene est le 2,3,3,3-tetrafluoropropene ou le 1,3,3,3-tetrafluoropropene.

Selon un mode de réalisation préféré, le récipient est un récipient fermé résistant à une pression d'épreuve, ladite pression d'épreuve étant comprise entre 10 et 100 bar, avantageusement entre 15 et 70 bar, de préférence entre 20 et 60 bar, en particulier de 40 à 50 bar.

Selon un mode de réalisation préféré, ledit récipient est de forme cylindrique et est monté au sein d'un cadre en acier, ledit cadre respectant les dimensions des iso containers selon les normes ISO 1496-1:2013.

Selon un second aspect, l'invention fournit une méthode pour le stockage d'une composition comprenant du tetrafluoropropene, ladite méthode comprenant la fourniture d'un récipient en métal dont la surface intérieure est au moins partiellement recouverte par une résine de type polyéther ou polyol ; et le remplissage dudit récipient par une composition comprenant du tetrafluoropropene.

Selon un mode de réalisation préféré, le récipient est en acier au carbone et le revêtement est une résine polyéther ou polyol selon l'invention ; et avantageusement le tetrafluoropropene est le 2,3,3,3-tetrafluoropropene ou le 1,3,3,3-tetrafluoropropene.

Selon un troisième aspect, l'invention fournit un dispositif pour charger un circuit de climatisation ou de réfrigération, ou pour remplacer un mélange réfrigérant contenu dans un circuit de climatisation ou un circuit de réfrigération, ledit dispositif comprenant un premier récipient selon l'invention et un second récipient comprenant un lubrifiant, avantageusement ledit dispositif comprend également une ou plusieurs conduites aptes à relier lesdits premier et second récipient au circuit de climatisation ou de réfrigération, de préférence le circuit de climatisation est un circuit de climatisation d'un véhicule.

### Description détaillée de l'invention

Selon un premier aspect de l'invention, un récipient est fourni. Le récipient peut être de n'importe quelle forme, cependant, le récipient est de préférence cylindrique. Le récipient comprend une surface intérieure. De préférence, le récipient contient une composition comprenant du tetrafluoropropene. Ledit récipient est de préférence en métal. Le récipient peut éventuellement être compartimenté, par exemple pour contenir dans chacun des compartiments des compositions comprenant différentes quantités en tetrafluoropropene ou pour contenir par exemple dans un compartiment une composition comprenant du tetrafluoropropene et dans un autre compartiment un autre composé, par exemple un lubrifiant ou un autre composé fluoré tel qu'un hydrofluorocarbure, un hydrochlorofluorocarbure ou un hydrochlorocarbure.

Le récipient peut être adapté pour le transport d'une composition comprenant du tetrafluoropropene et/ou pour le stockage d'une composition comprenant du tetrafluoropropene. Cette dernière peut par exemple être issue d'une unité de production du 2,3,3,3-tetrafluoropropene ou du 1,3,3,3-tetrafluoropropene.

Le récipient peut être muni d'une ou plusieurs vannes permettant le remplissage et/ou la vidange de celui-ci. Par exemple, une vanne d'entrée peut être positionnée pour que le récipient soit alimenté avec un flux contenant une composition comprenant du tetrafluoropropene. Le récipient peut également être équipé d'une ou plusieurs vannes de sortie permettant de vider ledit récipient de la composition qu'il contient. Par exemple, la composition peut être vidée du récipient pour alimenter un dispositif de purification de la composition comprenant du tetrafluoropropene ou pour alimenter un second récipient selon la présente invention et adapté au transport d'une composition comprenant du tetrafluoropropene. La composition peut également être vidée du récipient pour alimenter un circuit de climatisation. Ainsi, le récipient selon la présente invention peut être une cuve de stockage d'une composition comprenant du tetrafluoropropene ou un container répondant aux normes de transport ISO. Ledit récipient est de préférence un récipient fermé résistant à la pression. Le récipient peut être une bouteille ou une cartouche ou un petit container résistant à la pression, dont le volume peut être de préférence inférieur à 1 m³, avantageusement inférieur à 0,1 m³, de préférence inférieur à 0,01 m³. Alternativement, le récipient peut être une cuve de stockage ou de transport dont le volume peut être supérieur à 0,5 m³, avantageusement supérieur à 100 m³, de préférence supérieure à 1000 m³, en particulier supérieur à 5000m³. De préférence, la cuve de stockage ou de transport peut être de forme sphérique ou cylindrique. Le récipient selon la présente invention peut comprendre une paroi isolante thermiquement.

De préférence, ladite surface intérieure du récipient en contact avec ladite composition comprenant du tetrafluoropropene est au moins partiellement recouverte par une résine de type polyéther ou polyol.

Selon un mode de réalisation préféré, au moins 90% de ladite surface intérieure en contact avec ladite composition peut être recouverte par une résine de type polyéther ou polyol, avantageusement au moins 95% de ladite surface intérieure en contact avec ladite composition peut être recouverte par une résine de type polyéther ou polyol, de préférence au moins 98% de ladite surface intérieure en contact avec ladite composition peut être recouverte par une résine de type polyéther ou polyol, en particulier au moins 99% de ladite surface intérieure en contact avec ladite composition peut être recouverte par une résine de type polyéther ou polyol. Plus particulièrement, toute la surface intérieure en contact avec ladite composition contenue dans ledit récipient peut être recouverte par une résine de type polyéther ou polyol. Ceci permet de minimiser les contacts entre ladite composition et l'acier. En effet, un contact prolongé entre la composition comprenant du tetrafluoropropene et l'acier, notamment le fer contenu dans l'acier, peut favoriser la formation d'hydrogène, notamment si la composition comprend de l'eau ou de l'acide chlorhydrique.

Selon un mode de réalisation préféré, la résine de type polyéther ou polyol est issue de monomères comprenant un groupement fonctionnel oxirane ou phénol.

Avantageusement, le groupement fonctionnel oxirane peut être lié à un ou plusieurs oxydes métalliques. L'oxyde métallique peut être la silice, l'oxyde d'aluminium, l'oxyde de magnésium, l'oxyde de titane, l'oxyde d'antimoine, l'oxyde de zinc, l'oxyde de fer ou l'oxyde de molybdène. De préférence, le groupement fonctionnel oxirane peut être lié à de l'oxyde d'aluminium ou de la silice.

Selon un mode de réalisation préféré, la résine de type polyéther ou polyol est issue de monomères comprenant un motif siloxirane.

De préférence, la résine de type polyéther ou polyol est issue de monomères comprenant un motif siloxirane de formule (I) 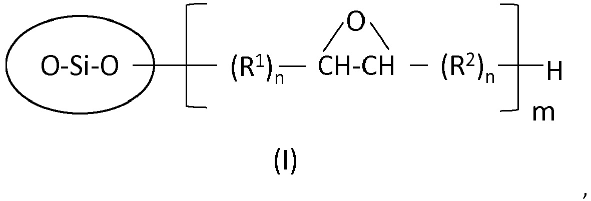 dans laquelle R¹ et R² sont indépendamment l'un de l'autre, et indépendamment pour chaque unité n et m, un groupement C₆-C₁₈ aryle, C₁-C₂₀ alkyle, C₃-C₂₀ cycloalkyle, C₃-C₂₀ cycloalkényle, C₂-C₂₀ alkényle, carbonyle de formule R³-C(O)-R⁴, ester de formule R³-C(O)-O-R⁴, éther de formule R³-O-R⁴; une amine de formule R³-N-R⁴, R² pouvant également être un groupement aldéhyde de formule R³-C(O)-H ; R³ et R⁴ étant choisis indépendamment l'un de l'autre parmi un groupement C₆-C₁₈ aryle, C₁-C₂₀ alkyle, C₃-C₂₀ cycloalkyle, C₃-C₂₀ cycloalkényle ou C₂-C₂₀ alkényle ; m est un nombre entier de 1 à 30, avantageusement de 1 à 20, de préférence de 5 à 10 ; et n est indépendamment pour R¹ et R² un nombre entier de 1 à 30, avantageusement de 1 à 20, de préférence de 5 à 10.

Le terme « alkyle » désigne un radical monovalent issu d'un alcane, linéaire ou branché, comprenant de 1 à 20 atomes de carbone. Le terme « cycloalkyle » désigne un radical monovalent issu d'un cycloalcane comprenant de 3 à 20 atomes de carbone. Le terme « aryle » désigne un radical monovalent issu d'un arène comprenant de 6 à 18 atomes de carbone. Le terme « alkényle » désigne un radical monovalent de 2 à 20 atomes de carbone et au moins une double liaison carbone-carbone. Le terme « halogène » se réfère à un groupement -F, -Cl, -Br ou -I. Le terme « cycloalkényle » se réfère à un radical monovalent issu d'un cycloalcène comprenant de 3 à 20 atomes de carbone. Les groupements alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ peuvent être substitués ou non par un ou plusieurs substituants -OH, halogène, -NR^{a}C(O)R^{b}, -C(O)NR^{a}R^{b}, - NR^{a}R^{b}, -OR^{a}, -CO₂R^{a}, -OC(O)OR^{a}, -OC(O)R^{a}, -C(O)H, -C(O)R^{a}, dans lequel R^{a} et R^{b} sont indépendamment l'un de l'autre hydrogène, alkyle en C₁-C₂₀ non substitué, alkényle en C₂-C₂₀ non substitué, cycloalkyle en C₃-C₂₀ non substitué, cycloalkényle en C₃-C₂₀ non substitué, aryle en C₆-C₁₈ non substitué. Dans les substituants -NR^{a}R^{b}, R^{a} et R^{b} peuvent former avec l'atome d'azote auquel ils sont rattachés un hétérocycle saturé ou insaturé, aromatique ou non, comprenant de 5 à 10 chaînons.

Selon un autre mode de réalisation préféré, la résine de type polyéther ou polyol est issue de condensats d'un composé **A** avec un composé **B**, le composé **A** étant un composé phénol substitué ou non et le composé **B** étant un composé de formule R⁵C(O)R⁶ dans laquelle R⁵ et R⁶ sont indépendamment hydrogène, C₁-C₂₀ alkyle, C₆-C₂₀ aryle, C₃-C₂₀ cycloalkyle, C₂-C₂₀ alkényle. Le composé phénol substitué peut être substitué par l'un quelconque des substituants mentionnés ci-dessus. De préférence, le composé **A** est un phénol non substitué. Avantageusement, le composé **B** étant un composé de formule R⁵C(O)R⁶ dans laquelle R⁵ et R⁶ sont indépendamment hydrogène, C₁-C₁₀ alkyle, C₆-C₁₀ aryle, C₃-C₁₀ cycloalkyle, C₂-C₁₀ alkényle. De préférence, le composé **B** étant un composé de formule R⁵C(O)R⁶ dans laquelle R⁵ et R⁶ sont indépendamment hydrogène, C₁-C₅ alkyle, C₆-C₁₀ aryle, C₃-C₆ cycloalkyle, C₂-C₅ alkényle. En particulier, le composé **B** étant un composé de formule R⁵C(O)R⁶ dans laquelle R⁵ et R⁶ sont l'hydrogène.

De préférence, le composé **A** est le phénol C₆H₅OH et le composé **B** est le formaldéhyde.

Selon un mode de réalisation préféré, le récipient peut être en acier. Avantageusement, le récipient est en acier au carbone ou en acier inoxydable. De préférence, le récipient est en acier au carbone.

Avantageusement, ladite composition comprend au moins 15% en poids de tetrafluoropropene sur base du poids total de la composition. De préférence, ladite composition comprend au moins 40% en poids de tetrafluoropropene sur base du poids total de la composition, plus préférentiellement au moins 60% en poids de tetrafluoropropene sur base du poids total de la composition, en particulier au moins 70% en poids de tetrafluoropropene sur base du poids total de la composition, plus particulièrement au moins 80% en poids de tetrafluoropropene sur base du poids total de la composition.

Selon un mode de réalisation particulier, ladite composition peut comprendre au moins 90% en poids de tetrafluoropropene sur base du poids total de la composition, avantageusement au moins 95% en poids sur base du poids total de la composition, de préférence au moins 98% en poids sur base du poids total de la composition, en particulier au moins 99,5% en poids sur base du poids total de la composition.

De préférence, le tetrafluoropropene peut être le 2,3,3,3-tetrafluoropropene et/ou le 1,3,3,3-tetrafluoropropene.

Ainsi, ladite composition peut comprendre au moins 15% en poids de 2,3,3,3-tetrafluoropropene sur base du poids total de la composition. De préférence, ladite composition comprend au moins 40% en poids de 2,3,3,3-tetrafluoropropene sur base du poids total de la composition, plus préférentiellement au moins 60% en poids de 2,3,3,3-tetrafluoropropene sur base du poids total de la composition, en particulier au moins 70% en poids de 2,3,3,3-tetrafluoropropene sur base du poids total de la composition, plus particulièrement au moins 80% en poids de 2,3,3,3-tetrafluoropropene sur base du poids total de la composition. Selon un mode de réalisation particulier, ladite composition peut comprendre au moins 90% en poids de 2,3,3,3-tetrafluoropropene sur base du poids total de la composition, avantageusement au moins 95% en poids de 2,3,3,3-tetrafluoropropene sur base du poids total de la composition, de préférence au moins 98% en poids de 2,3,3,3-tetrafluoropropene sur base du poids total de la composition, en particulier au moins 99,5% en poids de 2,3,3,3-tetrafluoropropene sur base du poids total de la composition.

Alternativement, ladite composition peut comprendre au moins 15% en poids de 1,3,3,3-tetrafluoropropene sur base du poids total de la composition. De préférence, ladite composition comprend au moins 40% en poids de 1,3,3,3-tetrafluoropropene sur base du poids total de la composition, plus préférentiellement au moins 60% en poids de 1,3,3,3-tetrafluoropropene sur base du poids total de la composition, en particulier au moins 70% en poids de 1,3,3,3-tetrafluoropropene sur base du poids total de la composition, plus particulièrement au moins 80% en poids de 1,3,3,3-tetrafluoropropene sur base du poids total de la composition. Selon un mode de réalisation particulier, ladite composition peut comprendre au moins 90% en poids de 1,3,3,3-tetrafluoropropene sur base du poids total de la composition, avantageusement au moins 95% en poids de 1,3,3,3-tetrafluoropropene sur base du poids total de la composition, de préférence au moins 98% en poids de 1,3,3,3-tetrafluoropropene sur base du poids total de la composition, en particulier au moins 99,5% en poids de 1,3,3,3-tetrafluoropropene sur base du poids total de la composition.

Ladite composition contenue dans le récipient peut comprendre moins de 10000 ppm en poids d'eau sur base du poids total de la composition, avantageusement moins de 5000 ppm en poids d'eau sur base du poids total de la composition, de préférence moins de 1000 ppm en poids d'eau sur base du poids total de la composition, plus préférentiellement moins de 500 ppm en poids d'eau sur base du poids total de la composition, en particulier moins de 100 ppm en poids d'eau sur base du poids total de la composition, plus particulièrement moins de 50 ppm en poids d'eau sur base du poids total de la composition.

Avantageusement, ladite composition peut comprendre de 0,01 à 500 ppm en poids d'eau sur base du poids total de la composition, de préférence de 0,05 à 250 ppm en poids d'eau sur base du poids total de la composition, en particulier de 0,1 ppm à 100 ppm en poids d'eau sur base du poids total de la composition, plus particulièrement de 1 à 20 ppm d'eau sur base du poids total de la composition.

Selon un mode de réalisation préféré, ladite composition peut comprendre une teneur en air gazeux de 0,1% à 5% en volume sur base du volume total de la phase gazeuse, avantageusement de 0,5% à 3%, de préférence de 0,01% à 2% en volume sur base du volume total de la phase gazeuse.

De préférence, ladite composition peut comprendre moins de 10000 ppm en poids d'eau sur base du poids total de la composition, avantageusement moins de 5000 ppm en poids d'eau sur base du poids total de la composition, de préférence moins de 1000 ppm en poids d'eau sur base du poids total de la composition, plus préférentiellement moins de 500 ppm en poids d'eau sur base du poids total de la composition, en particulier moins de 100 ppm en poids d'eau sur base du poids total de la composition, plus particulièrement moins de 50 ppm en poids d'eau sur base du poids total de la composition ; et une teneur en air gazeux de 0,1% à 5% en volume sur base du volume total de la phase gazeuse, avantageusement de 0,5% à 3% en volume sur base du volume total de la phase gazeuse, de préférence de 0,01% à 2% en volume sur base du volume total de la phase gazeuse.

En particulier, ladite composition peut comprendre de 0,01 à 500 ppm en poids d'eau sur base du poids total de la composition, de préférence de 0,05 à 250 ppm en poids d'eau sur base du poids total de la composition, en particulier de 0,1 ppm à 100 ppm en poids d'eau sur base du poids total de la composition, plus particulièrement de 1 à 20 ppm en poids d'eau sur base du poids total de la composition ; et une teneur en air gazeux de 0,1% à 5% en volume sur base du volume total de la phase gazeuse, avantageusement de 0,5% à 3% en volume sur base du volume total de la phase gazeuse, de préférence de 0,01% à 2% en volume sur base du volume total de la phase gazeuse.

Selon un mode de réalisation préféré, la composition peut avoir une teneur en acide, calculée en équivalent d'acide chlorhydrique, inférieure à 100 ppm en poids d'acide sur base du poids total de la composition, avantageusement inférieure à 50 ppm en poids d'acide sur base du poids total de la composition, de préférence inférieure à 10 ppm en poids d'acide sur base du poids total de la composition, en particulier de 0,01 ppm à 2 ppm en poids d'acide sur base du poids total de la composition.

Avantageusement, ladite composition peut avoir une teneur en acide, calculée en équivalent d'acide chlorhydrique, inférieure à 100 ppm en poids sur base du poids total de la composition, avantageusement inférieure à 50 ppm en poids sur base du poids total de la composition, de préférence inférieure à 10 ppm en poids sur base du poids total de la composition, en particulier de 0,01 ppm à 2 ppm en poids sur base du poids total de la composition; et peut comprendre moins de 10000 ppm en poids d'eau sur base du poids total de la composition, avantageusement moins de 5000 ppm en poids d'eau sur base du poids total de la composition , de préférence moins de 1000 ppm en poids d'eau sur base du poids total de la composition , plus préférentiellement moins de 500 ppm en poids d'eau sur base du poids total de la composition, en particulier moins de 100 ppm en poids d'eau sur base du poids total de la composition, plus particulièrement moins de 50 ppm en poids d'eau sur base du poids total de la composition. Alternativement, ladite composition peut avoir une teneur en acide, calculée en équivalent d'acide chlorhydrique, inférieure à 100 ppm en poids sur base du poids total de la composition, avantageusement inférieure à 50 ppm en poids sur base du poids total de la composition, de préférence inférieure à 10 ppm en poids sur base du poids total de la composition, en particulier de 0,01 ppm à 2 ppm en poids sur base du poids total de la composition ; et peut comprendre de 0,01 à 500 ppm d'eau en poids sur base du poids total de la composition, de préférence de 0,05 à 250 ppm en poids sur base du poids total de la composition, en particulier de 0,1 ppm à 100 ppm en poids sur base du poids total de la composition, plus particulièrement de 1 à 20 ppm en poids sur base du poids total de la composition.

Ladite composition peut avoir une teneur en acide, calculée en équivalent d'acide chlorhydrique, inférieure à 100 ppm en poids sur base du poids total de la composition, avantageusement inférieure à 50 ppm en poids sur base du poids total de la composition, de préférence inférieure à 10 ppm en poids sur base du poids total de la composition, en particulier de 0,01 ppm à 2 ppm en poids sur base du poids total de la composition ; et une teneur en air gazeux de 0,1% à 5% en volume sur base du volume total de la phase gazeuse, avantageusement de 0,5% à 3% en volume sur base du volume total de la phase gazeuse, de préférence de 0,01% à 2% en volume sur base du volume total de la phase gazeuse.

De préférence, ladite composition peut avoir une teneur en acide, calculée en équivalent d'acide chlorhydrique, inférieure à 100 ppm en poids sur base du poids total de la composition, avantageusement inférieure à 50 ppm en poids sur base du poids total de la composition, de préférence inférieure à 10 ppm en poids sur base du poids total de la composition, en particulier de 0,01 ppm à 2 ppm en poids sur base du poids total de la composition ; et une teneur en air gazeux de 0,1% à 5% en volume sur base du volume total de la phase gazeuse, avantageusement de 0,5% à 3% en volume sur base du volume total de la phase gazeuse, de préférence de 0,01% à 2% en volume sur base du volume total de la phase gazeuse ; et peut comprendre de 0,01 à 500 ppm en poids d'eau sur base du poids total de la composition, de préférence de 0,05 à 250 ppm en poids d'eau sur base du poids total de la composition, en particulier de 0,1 ppm à 100 ppm en poids d'eau sur base du poids total de la composition, plus particulièrement de 1 à 20 ppm en poids d'eau sur base du poids total de la composition.

De préférence, ladite composition peut avoir une teneur en acide, calculée en équivalent d'acide chlorhydrique, inférieure à 100 ppm en poids sur base du poids total de la composition, avantageusement inférieure à 50 ppm en poids sur base du poids total de la composition, de préférence inférieure à 10 ppm en poids sur base du poids total de la composition, en particulier de 0,01 ppm à 2 ppm en poids sur base du poids total de la composition ; et une teneur en air gazeux de 0,1% à 5% en volume sur base du volume total de la phase gazeuse, avantageusement de 0,5% à 3% en volume sur base du volume total de la phase gazeuse, de préférence de 0,01% à 2% en volume sur base du volume total de la phase gazeuse ; et peut comprendre moins de 10000 ppm en poids d'eau sur base du poids total de la composition, avantageusement moins de 5000 ppm en poids d'eau sur base du poids total de la composition, de préférence moins de 1000 ppm en poids d'eau sur base du poids total de la composition, plus préférentiellement moins de 500 ppm en poids d'eau sur base du poids total de la composition, en particulier moins de 100 ppm en poids d'eau sur base du poids total de la composition, plus particulièrement moins de 50 ppm en poids d'eau sur base du poids total de la composition.

Ledit récipient peut être un récipient fermé résistant à une pression d'épreuve, ladite pression d'épreuve peut être comprise entre 10 et 100 bar, avantageusement entre 15 et 70 bar, de préférence entre 20 et 60 bar, en particulier entre 40 et 50 bar.

De préférence, lorsque ladite composition est contenue dans le récipient, celle-ci peut être composée d'une phase gazeuse et d'une phase liquide. En particulier, la phase gazeuse et la phase liquide peuvent avoir la même composition chimique, c'est-à-dire que la proportion de tetrafluoropropene dans la phase liquide et dans la phase gazeuse est substantiellement identique, lorsque ladite composition comprend au moins 95% en poids de tetrafluoropropene, de préférence au moins 98% en poids de tetrafluoropropene, en particulier au moins 99,5% en poids de tetrafluoropropene.

Selon un mode de réalisation préféré, ledit récipient est de forme cylindrique et est monté au sein d'un cadre en acier, ledit cadre respectant les dimensions des iso containers selon les normes ISO 1496-1:2013.

Selon un autre mode de réalisation préféré, ledit récipient est une cartouche résistant à ladite pression d'épreuve mentionnée ci-dessus. De préférence, le volume interne de la cartouche contenant une composition comprenant du 2,3,3,3-tetrafluoropropene ou du 1,3,3,3-tetrafluoropropene est inférieur à 1 m³, avantageusement inférieur à 0,1 m³, de préférence inférieur à 0,01 m³.

Selon un second aspect, l'invention fournit une méthode pour le stockage d'une composition comprenant du tetrafluoropropene, ladite méthode comprenant la fourniture d'un récipient en métal dont la surface intérieure est au moins partiellement recouverte par une résine de type polyéther ou polyol ; et le remplissage dudit récipient par une composition comprenant du tetrafluoropropene. Le récipient et la composition sont tels que définis dans le premier aspect de la présente invention.

Selon un mode de réalisation préféré, le récipient est en acier au carbone et la résine est telle que définie ci-dessus dans la description détaillée de l'invention, et avantageusement le tetrafluoropropene est le 2,3,3,3-tetrafluoropropene et/ou le 1,3,3,3-tetrafluoropropene.

Selon un autre aspect de la présente invention, un dispositif est fourni. Ledit dispositif comprend un récipient, de préférence une bouteille ou une cartouche ou un petit container, selon la présente invention et un second récipient comprenant un lubrifiant. Le dispositif selon l'invention peut ainsi être adapté à la préparation d'un mélange réfrigérant comprenant un lubrifiant et du tetrafluoropropene. Le dispositif selon l'invention peut aussi être adapté à la charge d'un circuit de climatisation, de préférence automobile, ou de réfrigération. De préférence, le lubrifiant peut être adapté à la climatisation automobile. A titre de lubrifiants on peut notamment utiliser des polyalkène glycols, des polyolesters et/ou des polyvinyléthers. Ledit dispositif comprend également des connecteurs reliant indépendamment ou non chacun des récipients à un récipient ou un circuit de climatisation ou de réfrigération dans lequel le lubrifiant dudit second récipient est mélangé à ladite composition comprenant du tetrafluoropropene contenue dans ledit récipient selon la présente invention.

### Exemples

Des coupons d'acier en carbone recouverts totalement d'une résine issue d'un monomère comprenant un groupement fonctionnel de type siloxirane ou d'une résine issue de condensats du phénol et du formaldéhyde sont introduits dans des tubes en verre scellés. On introduit par pesée dans chaque tube 5000 ppm d'acide chlorhydrique 0,1N. Le tube scellé est placé dans la carboglace pour faciliter le tirage et le transfert du fluide frigorigène dans celui-ci. La bouteille contenant le fluide frigorigène est raccordée au tube scellé, l'ensemble du dispositif est tiré au vide et l'étanchéité est vérifiée. Le fluide est ensuite détendu dans une bouteille tampon de volume connu. On peut ainsi introduire une masse déterminée du fluide dans le tube scellé. Cette masse est calculée en fonction de la différence de pression, de la température et du volume tampon. On vérifie à l'aide d'un manomètre que le produit a bien été transféré, et on scelle le tube. Le tube ainsi scellé est pesé puis placé dans une gaine métallique, et mis à l'étuve à 85°C pendant 14 jours ou pendant 1 mois. Dans cet exemple, le fluide frigorigène est le 2,3,3,3-tetrafluoropropene.

L'observation visuelle des tubes ne montre pas de changement de couleur du 2,3,3,3-tetrafluoropropene liquide que ce soit après 14 jours ou 1 mois. Aucun coproduit n'est détecté dans le fluide frigorigène. La résine issue d'un monomère comprenant un groupement fonctionnel de type siloxirane montre après un mois une légère coloration. La résine issue de condensats du phénol avec le formaldéhyde, quant à elle, ne montre aucun changement d'aspect après 1 mois. Un récipient revêtu d'une résine issue de condensats du phénol avec le formaldéhyde permet le stockage ou le transport de tetrafluoropropene de manière prolongée sans dégradation.

## Revendications

1. Récipient contenant une composition comprenant du tetrafluoropropene, ledit récipient étant en métal et comprenant une surface intérieure, ladite surface intérieure en contact avec ladite composition étant au moins partiellement recouverte par une résine de type polyéther ou polyol, avantageusement ladite composition comprend au moins 15% en poids de tetrafluoropropene sur base du poids total de la composition.

2. Récipient selon la revendication précédente **caractérisé en ce que** la résine de type polyéther ou polyol est issue de monomères comprenant un groupement fonctionnel oxirane ou phénol.

3. Récipient selon l'une quelconque des revendications précédentes **caractérisé en ce que** la résine de type polyéther ou polyol est issue de monomères comprenant un motif siloxirane.

4. Récipient selon la revendication précédente **caractérisé en ce que** la résine de type polyéther ou polyol est issue de monomères comprenant un motif siloxirane de formule (I) 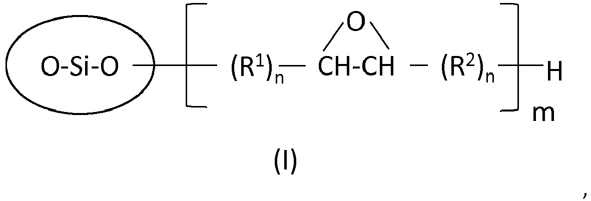
dans laquelle R¹ et R² sont indépendamment l'un de l'autre, et indépendamment pour chaque unité n et m, un groupement C₆-C₁₈ aryle, C₁-C₂₀ alkyle, C₃-C₂₀ cycloalkyle, C₃-C₂₀ cycloalkényle, C₂-C₂₀ alkényle, carbonyle de formule R³-C(O)-R⁴, ester de formule R³-C(O)-O-R⁴, éther de formule R³-O-R⁴ ; une amine de formule R³-N-R⁴, R² pouvant également être un groupement aldéhyde de formule R³-C(O)-H ; R³ et R⁴ étant choisis indépendamment l'un de l'autre parmi un groupement C₆-C₁₈ aryle, C₁-C₂₀ alkyle, C₃-C₂₀ cycloalkyle, C₃-C₂₀ cycloalkényle ou C₂-C₂₀ alkényle ;
m est un nombre entier de 1 à 30, avantageusement de 1 à 20, de préférence de 5 à 10 ; et
n est indépendamment pour R¹ et R² un nombre entier de 1 à 30, avantageusement de 1 à 20, de préférence de 5 à 10.

5. Récipient selon l'une quelconque des revendications 1 ou 2 **caractérisé en ce que** la résine de type polyéther ou polyol est issue de condensats d'un composé **A** avec un composé **B**, le composé **A** étant un composé phénol substitué ou non et le composé **B** étant un composé de formule R⁵C(O)R⁶ dans laquelle R⁵ et R⁶ sont indépendamment hydrogène, C₁-C₂₀ alkyle, C₆-C₂₀ aryle, C₃-C₂₀ cycloalkyle, C₂-C₂₀ alkényle.

6. Récipient selon la revendication précédente **caractérisé en ce que** le composé **A** est le phénol C₆H₅OH et le composé **B** est le formaldéhyde.

7. Récipient selon l'une quelconque des revendications précédentes **caractérisé en ce qu'**au moins 90% de ladite surface intérieure en contact avec ladite composition est recouverte par ladite résine de type polyéther ou polyol, avantageusement au moins 95% de ladite surface intérieure en contact avec ladite composition est recouverte par ladite résine de type polyéther ou polyol, de préférence au moins 98% de ladite surface intérieure en contact avec ladite composition est recouverte par ladite résine de type polyéther ou polyol, en particulier au moins 99% de ladite surface intérieure en contact avec ladite composition est recouverte par ladite résine de type polyéther ou polyol, plus particulièrement toute la surface intérieure du récipient en contact avec ladite composition est recouverte par ladite résine de type polyéther ou polyol.

8. Récipient selon l'une quelconque des revendications précédentes **caractérisé en ce que** le récipient est en acier.

9. Récipient selon l'une quelconque des revendications précédentes **caractérisé en ce que** le récipient est en acier au carbone.

10. Récipient selon l'une quelconque des revendications précédentes **caractérisé en ce que** la composition comprend moins de 10000 ppm en poids d'eau sur base du poids total de la composition, avantageusement moins de 5000 ppm en poids d'eau sur base du poids total de la composition, de préférence moins de 1000 ppm en poids d'eau sur base du poids total de la composition, en particulier de 0,1 ppm à 100 ppm en poids d'eau sur base du poids total de la composition, plus particulièrement de 1 à 20 ppm en poids d'eau sur base du poids total de la composition.

11. Récipient selon l'une quelconque des revendications précédentes **caractérisé en ce que** la composition est composée d'une phase gazeuse et d'une phase liquide.

12. Récipient selon la revendication précédente **caractérisé en ce que** la composition comprend une teneur en air gazeux de 0,1% à 5% en volume sur base du volume total de la phase gazeuse, avantageusement de 0,5% à 3% en volume sur base du volume total de la phase gazeuse, de préférence de 0,01% à 2% en volume sur base du volume total de la phase gazeuse.

13. Récipient selon l'une quelconque des revendications précédentes **caractérisé en ce que** la composition a une teneur en acide, calculée en équivalent d'acide chlorhydrique, inférieure à 100 ppm en poids sur base du poids total de la composition, avantageusement inférieure à 50 ppm en poids sur base du poids total de la composition, de préférence inférieure à 10 ppm en poids sur base du poids total de la composition, en particulier de 0,01 ppm à 2 ppm en poids sur base du poids total de la composition.

14. Récipient selon l'une quelconque des revendications précédentes **caractérisé en ce que** la composition comprend au moins 90% en poids de tetrafluoropropene sur base du poids total de la composition, avantageusement au moins 95% en poids de tetrafluoropropene sur base du poids total de la composition, de préférence au moins 98% en poids de tetrafluoropropene sur base du poids total de la composition, en particulier au moins 99,5% en poids de tetrafluoropropene sur base du poids total de la composition.

15. Récipient selon l'une quelconque des revendications précédentes **caractérisé en ce que** le tetrafluoropropene est le 2,3,3,3-tetrafluoropropene ou le 1,3,3,3-tetrafluoropropene.

16. Récipient selon l'une quelconque des revendications précédentes **caractérisé en ce que** le récipient est un récipient fermé résistant à une pression d'épreuve, ladite pression d'épreuve étant comprise entre 10 et 100 bar, avantageusement entre 15 et 70 bar, de préférence entre 20 et 60 bar, en particulier de 40 à 50 bar.

17. Récipient selon l'une quelconque des revendications précédentes **caractérisé en ce que** ledit récipient est de forme cylindrique et est monté au sein d'un cadre en acier, ledit cadre respectant les dimensions des iso containers selon les normes ISO 1496-1:2013.

18. Méthode pour le stockage d'une composition comprenant du tetrafluoropropene, ladite méthode comprenant la fourniture d'un récipient en métal dont la surface intérieure est au moins partiellement recouverte par une résine de type polyéther ou polyol ; et le remplissage dudit récipient par une composition comprenant du tetrafluoropropene.

19. Méthode selon la revendication précédente **caractérisée en ce que** le récipient est en acier au carbone et le revêtement est une résine telle que définie dans l'une quelconque des revendications 2 à 6 ; et avantageusement le tetrafluoropropene est le 2,3,3,3-tetrafluoropropene ou le 1,3,3,3-tetrafluoropropene.

20. Dispositif pour charger un circuit de climatisation ou pour remplacer un mélange réfrigérant contenu dans un circuit de climatisation ou de réfrigération, ledit dispositif comprenant un premier récipient selon l'une quelconque des revendications 1 à 13 et un second récipient comprenant un lubrifiant, avantageusement ledit dispositif comprend également une ou plusieurs conduites aptes à relier lesdits premier et second récipient au circuit de climatisation ou de réfrigération, de préférence le circuit de climatisation est un circuit de climatisation d'un véhicule.

## Patentansprüche

1. Behälter, enthaltend eine Zusammensetzung, die Tetrafluorpropen umfasst, wobei der Behälter aus Metall besteht und eine Innenoberfläche aufweist, wobei die mit der Zusammensetzung in Kontakt kommende Innenoberfläche zumindest teilweise mit einem Harz vom Polyether- oder Polyol-Typ bedeckt ist, vorteilhafterweise die Zusammensetzung mindestens 15 Gew.-% Tetrafluorpropen, bezogen auf das Gesamtgewicht der Zusammensetzung, umfasst.

2. Behälter nach dem vorhergehenden Anspruch, **dadurch gekennzeichnet, dass** sich das Harz vom Polyether- oder Polyol-Typ von Monomeren mit einer Oxiran- oder Phenolfunktion ableitet.

3. Behälter nach einem der vorhergehenden Ansprüche, **dadurch gekennzeichnet, dass** sich das Harz vom Polyether- oder Polyol-Typ von Monomeren mit einer Siloxiran-Einheit ableitet.

4. Behälter nach dem vorhergehenden Anspruch, **dadurch gekennzeichnet, dass** sich das Harz vom Polyether- oder Polyol-Typ von Monomeren mit einer Siloxiran-Einheit der Formel (I) 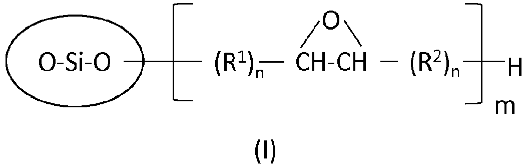
ableitet, in der R¹ und R² unabhängig voneinander und unabhängig für jede Einheit n und m für ein C₆-C₁₈-Aryl-, C₁-C₂o-Alkyl-, C₃-C₂₀-Cycloalkyl-, C₃-C₂₀-Cycloalkenyl-, C₂-C₂₀-Alkenylgruppe, eine Carbonylgruppe der Formel R³-C(O)-R⁴, eine Estergruppe der Formel R³-C(O)-O-R⁴, eine Ethergruppe der Formel R³-O-R⁴, oder ein Amin der Formel R³-N-R⁴ stehen, wobei R² auch für eine Aldehydgruppe der Formel R³-C(O)-H stehen kann; wobei R³ und R⁴ unabhängig voneinander aus einer C₆-C₁₈-Aryl-, C₁-C₂₀-Alkyl-, C₃-C₂₀-Cycloalkyl-, C₃-C₂₀-Cycloalkenyl- oder C₂-C₂₀-Alkenylgruppe ausgewählt sind;
m für eine ganze Zahl von 1 bis 30, vorteilhafterweise von 1 bis 20, vorzugsweise von 5 bis 10, steht und
n unabhängig für R¹ und R² für eine ganze Zahl von 1 bis 30, vorteilhafterweise von 1 bis 20, vorzugsweise von 5 bis 10, steht.

5. Behälter nach einem der Ansprüche 1 oder 2, **dadurch gekennzeichnet, dass** sich das Harz vom Polyether- oder Polyol-Typ von Kondensaten einer Verbindung **A** mit einer Verbindung **B** ableitet, wobei es sich bei der Verbindung **A** um eine gegebenenfalls substituierte Phenolverbindung handelt und es sich bei der Verbindung **B** um eine Verbindung der Formel R⁵C(O)R⁶ handelt, wobei R⁵ und R⁶ unabhängig für Wasserstoff, C₁-C₂₀-Alkyl, C₆-C₂₀-Aryl, C₃-C₂₀-Cycloalkyl oder C₂-C₂₀-Alkenyl stehen.

6. Behälter nach dem vorhergehenden Anspruch, **dadurch gekennzeichnet, dass** es sich bei der Verbindung **A** um Phenol C₆H₅OH handelt und es sich bei Verbindung **B** um Formaldehyd handelt.

7. Behälter nach einem der vorhergehenden Ansprüche, **dadurch gekennzeichnet, dass** mindestens 90 % der mit der Zusammensetzung in Kontakt kommenden Innenoberfläche mit dem Harz vom Polyether- oder Polyol-Typ bedeckt sind, vorteilhafterweise mindestens 95 % der mit der Zusammensetzung in Kontakt kommenden Innenoberfläche mit dem Harz vom Polyether- oder Polyol-Typ bedeckt sind, vorzugsweise mindestens 98 % der mit der Zusammensetzung in Kontakt kommenden Innenoberfläche mit dem Harz vom Polyether- oder Polyol-Typ bedeckt sind, insbesondere mindestens 99 % der mit der Zusammensetzung in Kontakt kommenden Innenoberfläche mit dem Harz vom Polyether- oder Polyol-Typ bedeckt sind, spezieller die gesamte mit der Zusammensetzung in Kontakt kommende Innenoberfläche des Behälters mit dem Harz vom Polyether- oder Polyol-Typ bedeckt ist.

8. Behälter nach einem der vorhergehenden Ansprüche, **dadurch gekennzeichnet, dass** der Behälter aus Stahl besteht.

9. Behälter nach einem der vorhergehenden Ansprüche, **dadurch gekennzeichnet, dass** der Behälter aus unlegiertem Stahl besteht.

10. Behälter nach einem der vorhergehenden Ansprüche, **dadurch gekennzeichnet, dass** die Zusammensetzung weniger als 10.000 Gew.-ppm Wasser, bezogen auf das Gesamtgewicht der Zusammensetzung, vorteilhafterweise weniger als 5000 Gew.-ppm Wasser, bezogen auf das Gesamtgewicht der Zusammensetzung, vorzugsweise weniger als 1000 Gew.-ppm Wasser, bezogen auf das Gesamtgewicht der Zusammensetzung, insbesondere 0,1 bis 100 Gew.-ppm Wasser, bezogen auf das Gesamtgewicht der Zusammensetzung, spezieller 1 bis 20 Gew.-ppm Wasser, bezogen auf das Gesamtgewicht der Zusammensetzung, umfasst.

11. Behälter nach einem der vorhergehenden Ansprüche, **dadurch gekennzeichnet, dass** die Zusammensetzung aus einer Gasphase und einer Flüssigphase besteht.

12. Behälter nach dem vorhergehenden Anspruch, **dadurch gekennzeichnet, dass** die Zusammensetzung einen Gehalt an gasförmiger Luft von 0,1 bis 5 Vol.-%, bezogen auf das Gesamtvolumen der Gasphase, vorteilhafterweise von 0,5 bis 3 Vol.-%, bezogen auf das Gesamtvolumen der Gasphase, vorzugsweise von 0,01 bis 2 Vol.-%, bezogen auf das Gesamtvolumen der Gasphase, umfasst.

13. Behälter nach einem der vorhergehenden Ansprüche, **dadurch gekennzeichnet, dass** die Zusammensetzung einen Säuregehalt, berechnet als Salzsäure-Äquivalent, von weniger als 100 Gew.-ppm, bezogen auf das Gesamtgewicht der Zusammensetzung, vorteilhafterweise weniger als 50 Gew.-ppm, bezogen auf das Gesamtgewicht der Zusammensetzung, vorzugsweise weniger als 10 Gew.-ppm, bezogen auf das Gesamtgewicht der Zusammensetzung, insbesondere von 0,01 bis 2 Gew.-ppm, bezogen auf das Gesamtgewicht der Zusammensetzung, aufweist.

14. Behälter nach einem der vorhergehenden Ansprüche, **dadurch gekennzeichnet, dass** die Zusammensetzung mindestens 90 Gew.-% Tetrafluorpropen, bezogen auf das Gesamtgewicht der Zusammensetzung, vorzugsweise mindestens 95 Gew.-% Tetrafluorpropen, bezogen auf das Gesamtgewicht der Zusammensetzung, vorzugsweise mindestens 98 Gew.-% Tetrafluorpropen, bezogen auf das Gesamtgewicht der Zusammensetzung, insbesondere mindestens 99,5 Gew.-% Tetrafluorpropen, bezogen auf das Gesamtgewicht der Zusammensetzung, umfasst.

15. Behälter nach einem der vorhergehenden Ansprüche, **dadurch gekennzeichnet, dass** es sich bei dem Tetrafluorpropen um 2,3,3,3-Tetrafluorpropen oder 1,3,3,3-Tetrafluorpropen handelt.

16. Behälter nach einem der vorhergehenden Ansprüche, **dadurch gekennzeichnet, dass** es sich bei dem Behälter um einen geschlossenen Behälter handelt, der einem Testdruck widersteht, wobei der Testdruck zwischen 10 und 100 bar, vorteilhafterweise zwischen 15 und 70 bar, vorzugsweise zwischen 20 und 60 bar, insbesondere im Bereich von 40 bis 50 bar, liegt.

17. Behälter nach einem der vorhergehenden Ansprüche, **dadurch gekennzeichnet, dass** der Behälter eine zylindrische Form aufweist und in einem Stahlrahmen montiert ist, wobei der Rahmen den Abmessungen von Iso-Containern gemäß den ISO-Normen 1496-1:2013 entspricht.

18. Verfahren zur Lagerung einer Zusammensetzung, die Tetrafluorpropen umfasst, wobei das Verfahren das Bereitstellen eines Metallbehälters, dessen Innenoberfläche zumindest teilweise mit einem Harz vom Polyether- oder Polyol-Typ bedeckt ist, und das Füllen des Behälters mit einer Zusammensetzung, die Tetrafluorpropen umfasst, umfasst.

19. Verfahren nach dem vorhergehenden Anspruch, **dadurch gekennzeichnet, dass** der Behälter aus unlegiertem Stahl besteht und es sich bei der Beschichtung um ein Harz gemäß einem der Ansprüche 2 bis 6 handelt und es sich vorteilhafterweise bei dem Tetrafluorpropen um 2,3,3,3-Tetrafluorpropen oder 1,3,3,3-Tetrafluorpropen handelt.

20. Vorrichtung zum Beschicken eines Klimatisierungskreislaufs oder zum Ersetzen einer in einem Klimatisierungs- oder Kältekreislauf enthaltenen Kältemittelmischung, wobei die Vorrichtung einen ersten Behälter nach einem der Ansprüche 1 bis 13 und einen zweiten Behälter, der ein Schmiermittel umfasst, aufweist, vorteilhafterweise die Vorrichtung außerdem eine oder mehrere Leitungen aufweist, mit denen der erste und zweite Behälter an den Klimatisierungs- oder Kältekreislauf angeschlossen werden können, vorzugsweise es sich bei dem Klimatisierungskreislauf um einen Klimatisierungskreislauf eines Fahrzeugs handelt.

## Claims

1. Container containing a composition comprising tetrafluoropropene, said container being made from metal and comprising an inner surface, said inner surface in contact with said composition being at least partially covered with a polyether- or polyol-type resin; advantageously, said composition comprises at least 15% by weight of tetrafluoropropene relative to the total weight of the composition.

2. Container according to the preceding claim, **characterized in that** the polyether- or polyol-type resin is derived from monomers comprising an oxirane or phenol functional group.

3. Container according to either one of the preceding claims, **characterized in that** the polyether- or polyol-type resin is derived from monomers comprising a siloxirane entity.

4. Container according to the preceding claim, **characterized in that** the polyether- or polyol-type resin is derived from monomers comprising a siloxirane entity of formula (I) 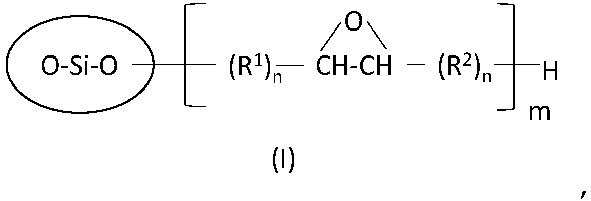
in which R¹ and R² are, independently of one another, and independently for each unit n and m, a C₆-C₁₈ aryl, C₁-C₂₀ alkyl, C₃-C₂₀ cycloalkyl, C₃-C₂₀ cycloalkenyl, or C₂-C₂₀ alkenyl group, a carbonyl group of formula R³-C(O)-R⁴, an ester group of formula R³-C(O)-O-R⁴, an ether group of formula R³-O-R⁴; or an amine of formula R³-N-R⁴, R² also being able to be an aldehyde group of formula R³-C(O)-H; R³ and R⁴ being chosen, independently of one another, from a C₆-C₁₈ aryl, C₁-C₂₀ alkyl, C₃-C₂₀ cycloalkyl, C₃-C₂₀ cycloalkenyl or C₂-C₂₀ alkenyl group;
m is an integer ranging from 1 to 30, advantageously from 1 to 20, preferably from 5 to 10; and
n is independently for R¹ and R² an integer from 1 to 30, advantageously from 1 to 20, preferably from 5 to 10.

5. Container according to either one of Claims 1 and 2, **characterized in that** the polyether- or polyol-type resin is derived from condensates of a compound **A** with a compound **B,** the compound **A** being an optionally substituted phenol compound and the compound **B** being a compound of formula R⁵C(O)R⁶ in which R⁵ and R⁶ are independently hydrogen, C₁-C₂₀ alkyl, C₆-C₂₀ aryl, C₃-C₂₀ cycloalkyl, or C₂-C₂₀ alkenyl.

6. Container according to the preceding claim, **characterized in that** the compound **A** is phenol C₆H₅OH and the compound **B** is formaldehyde.

7. Container according to any one of the preceding claims, **characterized in that** at least 90% of said inner surface in contact with said composition is covered with said polyether- or polyol-type resin, advantageously at least 95% of said inner surface in contact with said composition is covered with said polyether- or polyol-type resin, preferably at least 98% of said inner surface in contact with said composition is covered with said polyether- or polyol-type resin, in particular at least 99% of said inner surface in contact with said composition is covered with said polyether- or polyol-type resin, more particularly the entire inner surface of the container in contact with said composition is covered with said polyether- or polyol-type resin.

8. Container according to any one of the preceding claims, **characterized in that** the container is made from steel.

9. Container according to any one of the preceding claims, **characterized in that** the container is made from carbon steel.

10. Container according to any one of the preceding claims, **characterized in that** the composition comprises less than 10 000 ppm by weight of water relative to the total weight of the composition, advantageously less than 5000 ppm by weight of water relative to the total weight of the composition, preferably less than 1000 ppm by weight of water relative to the total weight of the composition, in particular from 0.1 ppm to 100 ppm by weight of water relative to the total weight of the composition, more particularly from 1 to 20 ppm by weight of water relative to the total weight of the composition.

11. Container according to any one of the preceding claims, **characterized in that** the composition is made up of a gas phase and a liquid phase.

12. Container according to the preceding claim, **characterized in that** the composition comprises a gaseous air content of from 0.1% to 5% by volume relative to the total volume of the gas phase, advantageously from 0.5% to 3% by volume relative to the total volume of the gas phase, preferably from 0.01% to 2% by volume relative to the total volume of the gas phase.

13. Container according to any one of the preceding claims, **characterized in that** the composition has an acid content, calculated in hydrochloric acid equivalent, of less than 100 ppm by weight relative to the total weight of the composition, advantageously less than 50 ppm by weight relative to the total weight of the composition, preferably less than 10 ppm by weight relative to the total weight of the composition, in particular from 0.01 ppm to 2 ppm by weight relative to the total weight of the composition.

14. Container according to any one of the preceding claims, **characterized in that** the composition comprises at least 90% by weight of tetrafluoropropene relative to the total weight of the composition, advantageously at least 95% by weight of tetrafluoropropene relative to the total weight of the composition, preferably at least 98% by weight of tetrafluoropropene relative to the total weight of the composition, in particular at least 99.5% by weight of tetrafluoropropene relative to the total weight of the composition.

15. Container according to any one of the preceding claims, **characterized in that** the tetrafluoropropene is 2,3,3,3-tetrafluoropropene or 1,3,3,3-tetrafluoropropene.

16. Container according to any one of the preceding claims, **characterized in that** the container is a closed container which withstands a test pressure, said test pressure being between 10 and 100 bar, advantageously between 15 and 70 bar, preferably between 20 and 60 bar, in particular from 40 to 50 bar.

17. Container according to any one of the preceding claims, **characterized in that** said container is cylindrical in shape and is mounted within a steel framework, said framework adhering to the dimensions of iso containers according to the standards ISO 1496-1:2013.

18. Method for storing a composition comprising tetrafluoropropene, said method comprising the provision of a metal container, the inner surface of which is at least partially covered with a polyether- or polyol-type resin; and the filling of said container with a composition comprising tetrafluoropropene.

19. Method according to the preceding claim, **characterized in that** the container is made from carbon steel and the coating is a resin as defined in any one of Claims 2 to 6, and the tetrafluoropropene is advantageously 2,3,3,3-tetrafluoropropene or 1,3,3,3-tetrafluoropropene.

20. Device for loading an air-conditioning circuit or for replacing a refrigerant mixture contained in an air-conditioning or refrigeration circuit, said device comprising a first container according to any one of Claims 1 to 13 and a second container comprising a lubricant; advantageously, said device also comprises one or more pipes capable of linking said first and second containers to the air-conditioning or refrigeration circuit; preferably, the air-conditioning circuit is an air-conditioning circuit of a vehicle.
